# EUROPEAN PATENT SPECIFICATION

(11) **EP 2 448 509 B1**
(45) Date of publication and mention of the grant of the patent: **22.10.2014**
(21) Application number: 10716200.0
(22) Date of filing: 22.04.2010
(51) Int. Cl.: A61B 18/14, A61B 18/00, A61B 18/18

(54) **BIPOLAR RESECTION DEVICE HAVING SIMPLIFIED ROTATIONAL CONTROL AND BETTER VISUALIZATION**
BIPOLARE RESEKTIONSVORRICHTUNG MIT VEREINFACHETER DREHSTEUERUNG UND BESSERER ANSICHT
DISPOSITIF DE RÉSECTION BIPOLAIRE AYANT UNE COMMANDE ROTATIONNELLE SIMPLIFIÉE ET UNE MEILLEURE VISUALISATION

(30) Priority: 30.06.2009 US 458064
(43) Date of publication of application: 09.05.2012
(73) Proprietor: Gyrus ACMI, Inc., Southborough, MA 01772 (US)
(72) Inventor: ST. GEORGE, Lawrence J., Sudbury, Massachusetts 01776 (US); DURAN, John S., Brewer, Maine 04412 (US); MANSFIELD, Richard P., Athol, Massachusetts 01331 (US)
(74) Representative: Wallin, Nicholas James
(86) International application number: PCT/US2010/032018
(87) International publication number: WO 2011/002545

(56) References cited:
- WO-A2-01/95810
- WO-A2-2007/031990
- US-A- 749 689
- US-A- 2 002 559
- US-A- 2 008 525
- US-B1- 6 730 081

## Description

### BACKGROUND

An improved bipolar resection device provides an intuitive finger grip control in a smaller sheath package. Bipolar electrode wires occupy less space by extending in a closely abutting relationship along the sheath and exiting in a stacked or one-above-the- other orientation that provides a reduced profile, allowing for better visualization during resection. In various embodiments, resection motion is provided through a rotational sweep transverse to the longitudinal axis of the resection device.

### PRIOR ART

WO 2007/031990 describes a dual-action actuating system, interconnecting an effecter with a single actuating mechanism activating the effecter in a sequence of rotational and linear maneuvers and a method of dual-action actuating. It also discloses a working tool comprising the dual-action system; the tool having a main longitudinal axis having a distal end and a proximal end; at least one effecter is located in the distal end, being in a communication with the single actuating mechanism, located at the proximal portion of the tool and a method of actuating a dual-action working tool. An endoscope having a dual-action actuating mechanism useful for manoeuvring at least one effecter located adjacent to a body portion to be treated in a sequence of rotational and linear maneuvers comprising a unified actuating module in communication with said effecter is also provided.

### SUMMARY

An enlarged prostate can inhibit the free flow of urine from the bladder and causes discomfort. Such an enlarged prostate requires some form of tissue reduction in order to improve urine flow. Various treatment methods exist for tissue reduction of an enlarged prostate. Known methods include, for example, heating of the prostate with very hot water, infrared or microwave radiation to "kill" tissue (which will then slough off); burning or vaporizing tissue directly with high energy lasers of various wavelengths; vaporizing tissue with a resecting device having energized electrodes of various shapes that are brought into close proximity or contact with the tissue; or resecting tissue with an energized loop electrode that cuts a strip of tissue at a time. Resecting electrodes are moved with the aid of a handheld tool (working element) that extends/retracts to provide a burning or cutting action on the tissue. The electrodes may be monopolar, in which the return current goes through the patient's body, or bipolar, in which the return current goes through the tissue between the electrodes, or through the single electrode alone and via RF energy creates burning or cutting action in close proximity to the electrode surface.

Heating of the prostate by hot water, infrared or microwave radiation requires fairly complex capital equipment devices. Additionally, the results in many cases may be unsatisfactory, and in others are not as effective as other methods.

Laser equipment can also be complex and expensive. Moreover, special safety equipment such as eye protection and warning signs are required. Depending on the wavelength used, sub-optimal results may be achieved in terms of tissue affected. However, fingertip control methods in some laser equipment have been found to be quite satisfactory in terms of operation.

Some electrodes, particularly bipolar ones, can produce satisfactory local tissue resection. However, current methods of operating the active component of the electrode with the working element require repetitive thumb "trigger" squeezing and wrist rotation, which can be cumbersome and fatiguing to the surgeon. Thus, the procedure is not completely satisfactory for the surgeon. Also, in general, electrodes and their generators are not designed for continuous operation, and instead operate in discrete "strokes." This increases procedure time. Aspects of the disclosure provide a finger grip control mechanism that results in simplified and improved control during resection that can achieve near continuous resection by a combination of lateral sweeps and longitudinal movement.

Another area where improvement can be made is in visualization. Resecting devices rely on optics to visualize the resection procedure. However, many resecting device electrode designs provide substantial impediments to the field of view to the surgeon.

Aspects of the disclosure provide various electrode assemblies that have a reduced obstruction to the field of view of a resecting site through the optics, while achieving satisfactory resection.

The invention is defined in appended claim 1. Preferred embodiments are described in the dependent claims.

In an exemplary arrangement, a resection device includes: a sheath having a proximal end and a distal end defining a longitudinal through bore therebetween, the distal end having a protruding insulated distal tip; a telescopic unit comprising a telescope extending from the proximal end of the sheath and optics extending from the telescope through the through bore and to the distal end of the sheath where the optics provide visualization of the resection tissue site, the optics extending longitudinally along the through bore; a bipolar electrode assembly extending from the proximal end of the sheath through the through bore substantially parallel to the optics, and to the distal end of the sheath, the bipolar electrode assembly including two electrode wires extending substantially parallel with the optics, the two electrode wires at least at the distal end of the sheath being oriented one above the other and defining a longitudinal axis parallel to the longitudinal through bore distal tips of the two bipolar electrode wires extending away from the optics at a non-zero angle relative to the longitudinal axis and being connected by an electrode oriented in the plane of the longitudinal axis; and a finger grip control mechanism provided external to the sheath and connected to the proximal end of the bipolar electrode wires to manipulate movement of the bipolar electrode assembly during resection by a sweeping rotary movement about the longitudinal axis. The rotary movement includes movement of the distal tips of the bipolar electrode wires between an insertion position where the distal tips are positioned within the sheath opposing the protruding insulating distal tip and a resection position rotated away from the insertion position where the distal tips are oriented outside of the sheath, resection being achieved in the resection position by the sweeping rotary movement about the longitudinal axis. The finger grip control mechanism is isolated from the telescopic unit such that rotation of the finger grip control mechanism is independent of rotation of the telescopic unit.

In various embodiments, the sheath may have an oval shape.

In certain embodiments, the bipolar electrode has a narrow cross-sectional profile to improve resection site visualization.

### BRIEF DESCRIPTION OF THE DRAWINGS

Figure 1 illustrates a front perspective view of an embodiment of a bipolar resection device with sheath having simplified rotational control and improved visualization;
Figure 2 illustrates a rear perspective view of the bipolar resection device of Figure 1;
Figure 3 illustrates a closeup perspective view of an exemplary bipolar button electrode at the tip of the sheath in a deployed position;
Figure 4 illustrates a closeup perspective view of the bipolar button electrode of Figure 3 in a retracted insertion/removal position;
Figure 5 illustrates a partial cross-sectional view of the button electrode at the tip of the sheath;
Figure 6 illustrates an end view of the tip of Figure 5;
Figure 7 is a partial perspective view of the tip of the button electrode of Fig. 3;
Figure 8 is a partial perspective view of the bipolar electrode of Figure 7 showing a central junction region where flexible and metal shaft regions mate;
Figures 9-10 illustrate closeup perspective views of an alternative bipolar electrode structure in the form of an ovoid electrode;
Figures 11-12 illustrate closeup perspective views of a further alternative bipolar electrode structure in the form of a narrow, wedge shape.
Figures 13-14 illustrate closeup perspective views of another alternative bipolar electrode structure in the form of a longitudinally oriented loop;
Figure 15 illustrates a side view of a conventional resection device;
Figure 16 illustrates an end view of the sheath tip of the conventional resection device of Figure 15 showing laterally separated bipolar electrode leads extending on either side of visualization optics;
Figure 17 illustrates a plan view of the bipolar electrode of Figures 15-16 showing the laterally separated electrodes;
Figure 18 illustrates a cross-sectional view of the conventional resection device of Figure 15; and
Figure 19 illustrates examples of various conventional electrode configurations.

### DETAILED DESCRIPTION OF EMBODIMENT

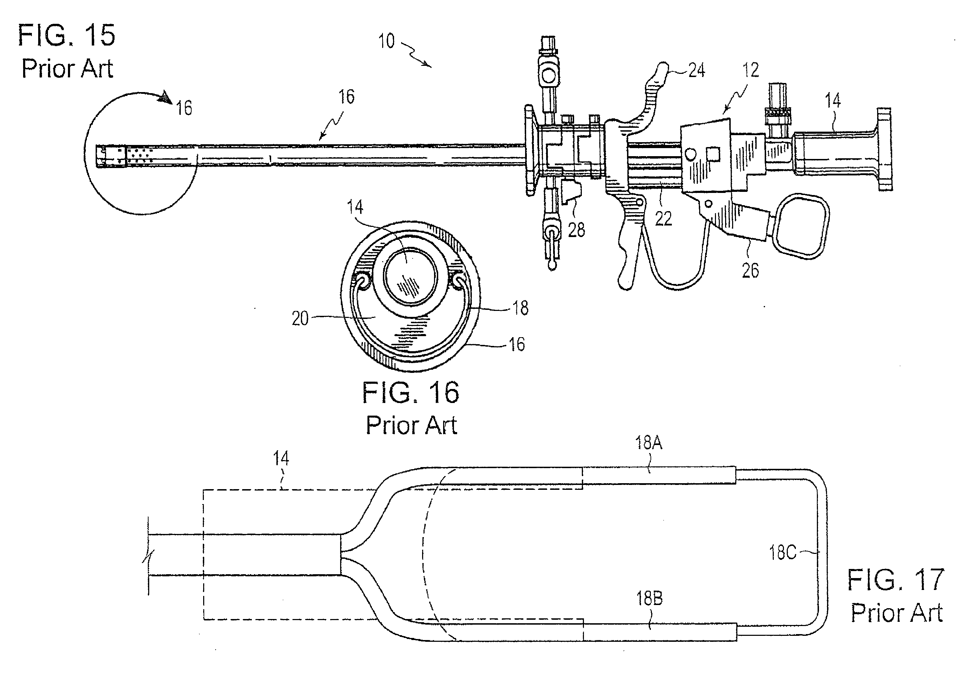

Conventional bipolar resecting devices 10, such as a resectoscope, are shown in Figs. 15-18. More specific examples of such resectoscopes may be found in U.S. Patents Nos. 6,712,759 to Muller (assigned to ACMI Corporation), U.S. Patent No. 7,118,569 to Snay et al. (assigned to ACMI Corporation), and U.S. Patent No. 6,827,717 to Brommersma et al. (assigned to Olympus Winter & Ibe GmbH).

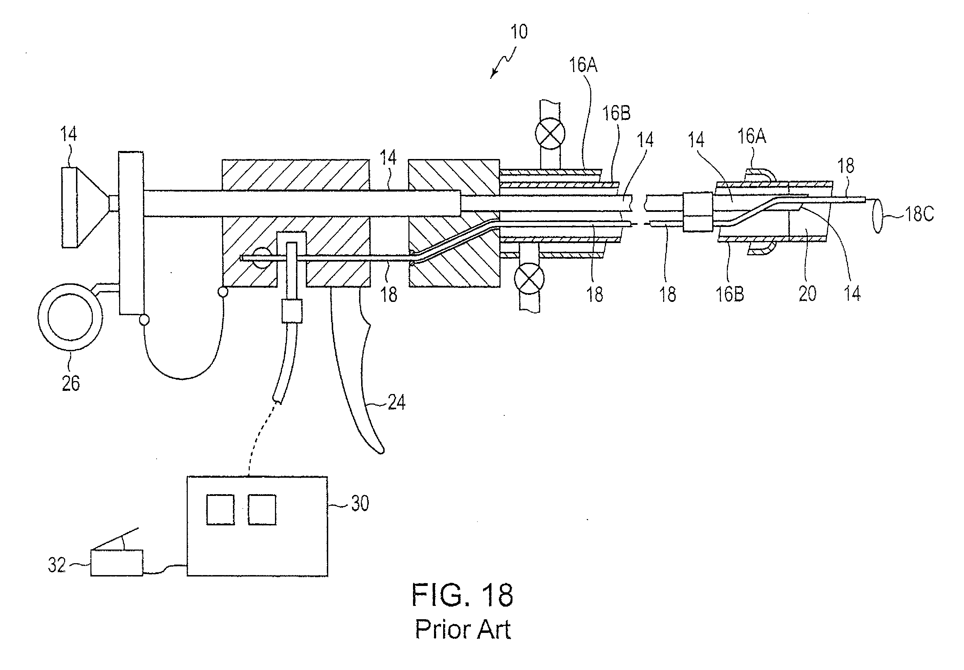

A conventional resecting device 10 includes a working element 12, a telescopic unit 14, a round sheath assembly 16 (inner and outer sheaths 16A, 16B in Fig. 18), and an electrode assembly 18 extending within a through bore 20 of the inner sheath. Visualization optics 14B of telescopic unit 14 also extend within through bore 20 and are connected to an eyepiece 14A of the telescopic unit 14 on the proximal end of the working element 12.

The working element 12 is attached to sheath 16 through a latch 28 and typically includes a frame 22, a front handle 24, and a moveable portion 26 having a thumb receiving aperture. The working element 12 is manipulated by squeezing of the front handle 24 and moveable portion 26 toward or away from each other by a predefined "stroke" to move the electrode assembly 18 in a movement direction, typically along the longitudinal axis of the sheath 16, to ablate or vaporize tissue.

The electrode assembly 18 is connected to a power generator 30 (Fig. 18) that can selectively apply power to the electrode assembly 18 in short bursts during the stroke of the working element 12 through use of a control pedal assembly 32.

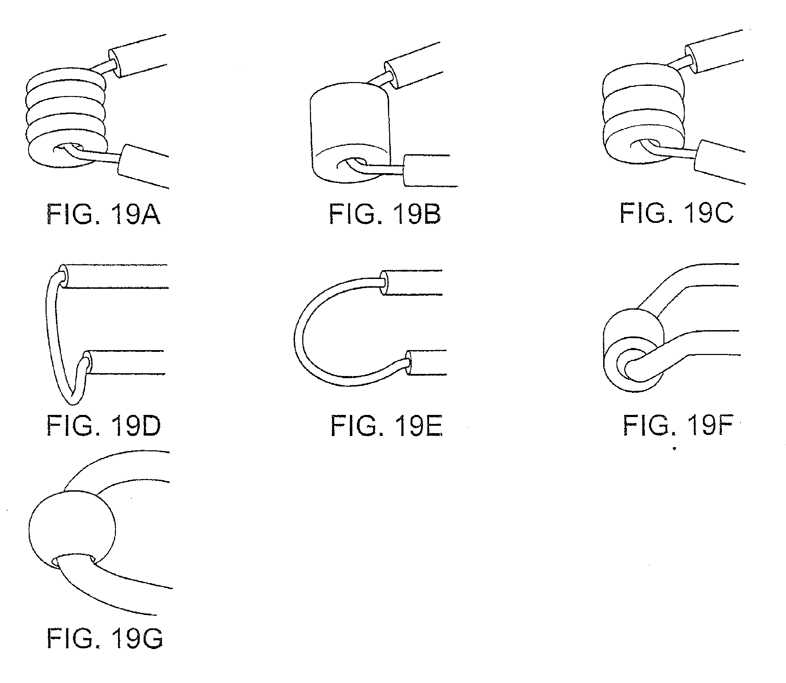

As better shown in Figs. 16-17, the electrode assembly 18, at least near a distal end of the sheath 16 separates into two electrode wires 18A, 18B provided on opposite sides of visualization optics 14. The electrode wires 18A, 18B connect through an electrode 18C, which is shown in the form of a loop. However, electrode 18C can take various other forms including various disks, loops, rollers or ball electrodes as shown in Fig. 19.

Further details of a conventional bipolar resectoscope can be seen in Fig. 18, where sheath 16 is shown to include an outer sheath 16A and an inner sheath 16B, both of a round cross-sectional shape. At the distal end of the sheath, the electrode wires 18A, 18B are oriented on opposite sides of the visualization optics 14 (better shown in Fig. 17 and also in Fig. 16).

In a bipolar configuration, one of the electrode wires 18A, 18B is an active power element and the other is a return element. Electrical energy is applied to a patient through the active power element and returns through the return element. Power is provided to the active element by the power generator 30 and the electrical circuit is completed by body tissue disposed in contact with the active element and return element (electrode wires 18A, 18B).

As mentioned above, movement of the electrode is typically through a translation of the distal end of the electrode assembly along the longitudinal axis of the sheath 16 by a "stroke" distance to resect or vaporize a resection site of body tissue. However, certain resectoscopes can also provide rotation by rotation of the entire working element 12 assembly, which rotates the telescopic unit 14 as well as inner sheath 16B. This rotation requires a corresponding rotation of the surgeon's arm when gripping the working element with a thumb and finger.

Although resection by such conventional resectoscopes can result in satisfactory results, there is room for improvement in the ergonomics of the motion control. For example, the repetitive thumb "trigger" squeezing and wrist and arm rotation can be cumbersome and fatiguing to the surgeon. Thus, the procedure is not completely satisfactory for the surgeon.

Also, in general, electrodes and their generators are not designed for continuous operation, and instead operate in discrete "strokes." This increases the procedure time. Thus, further efficiencies can be provided.

Improvements in visualization and minimization of the incision size needed would be beneficial. However, due to the orientation of the electrode wires 18A, 18B on both sides of the visualization optics 14 and the downward extending electrode 18C, further reduction in the size of the sheath 16 in the current design is not feasible and is essentially limited to a round sheath of about _9mm or about 28 French (a measure of the circumference, or more specifically the path around the outside of a sheath that a taut thread or string would follow). _. Also, due to this configuration, further improvements in visualization are also limited as a fairly large cross-section of the electrode is provided in line with the visualization optics.

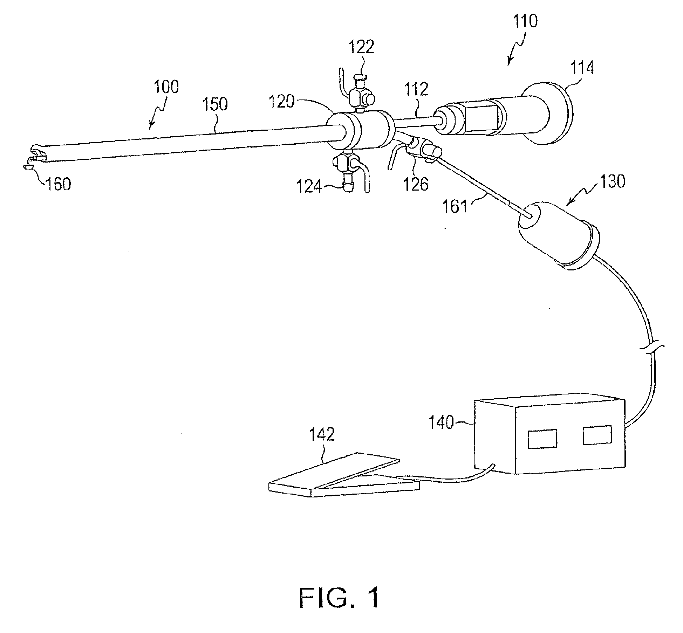

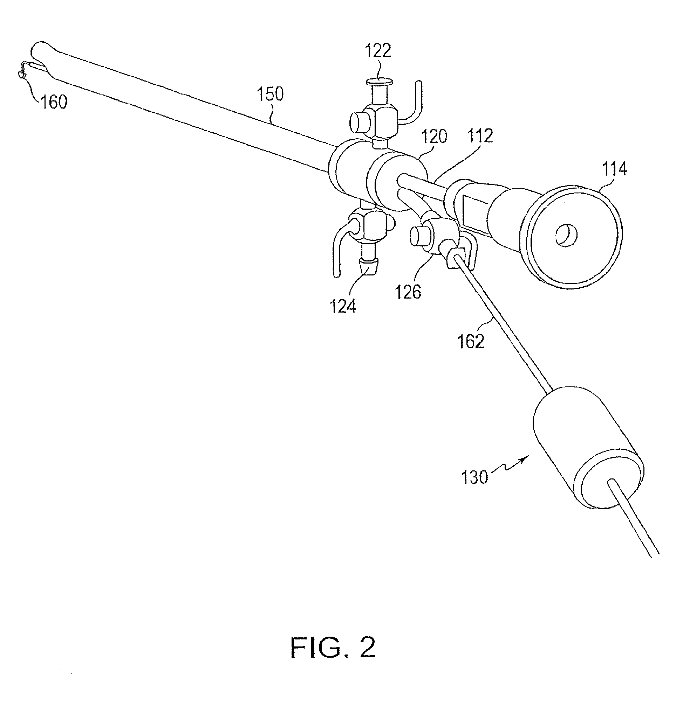

In exemplary embodiments, one or more of the above problems may be overcome by an improved resection device. An exemplary embodiment of an improved resection device is shown in Figs. 1-6. The resection device 100 includes a telescopic unit 110, a connection part 120, a finger grip control mechanism 130, a power generator unit 140, a sheath 150, and a bipolar electrode assembly 160.

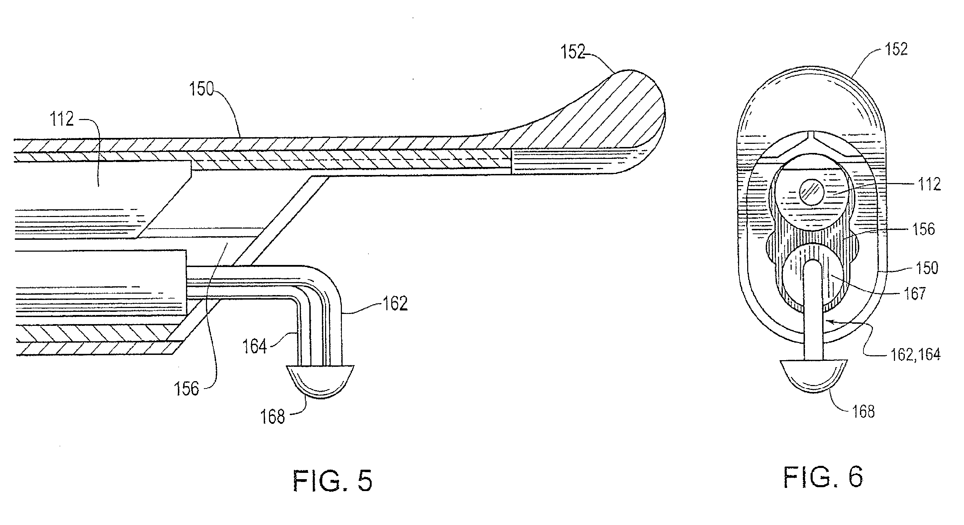

Telescopic unit 110 includes a telescope optics guide tube 112, a telescope eyepiece 114 and optics 116 (Figs. 5-6), such as fiber optics, which extend from eyepiece 114, through guide tube 122 towards sheath 150. Connection part 120 includes an inlet port 122, an outlet port 124 and a working tool guide tube 126. The working tool guide tube 126 includes an opening sized to receive a working tool component, such as a flexible shaft 161 of electrode assembly 160, therethrough.

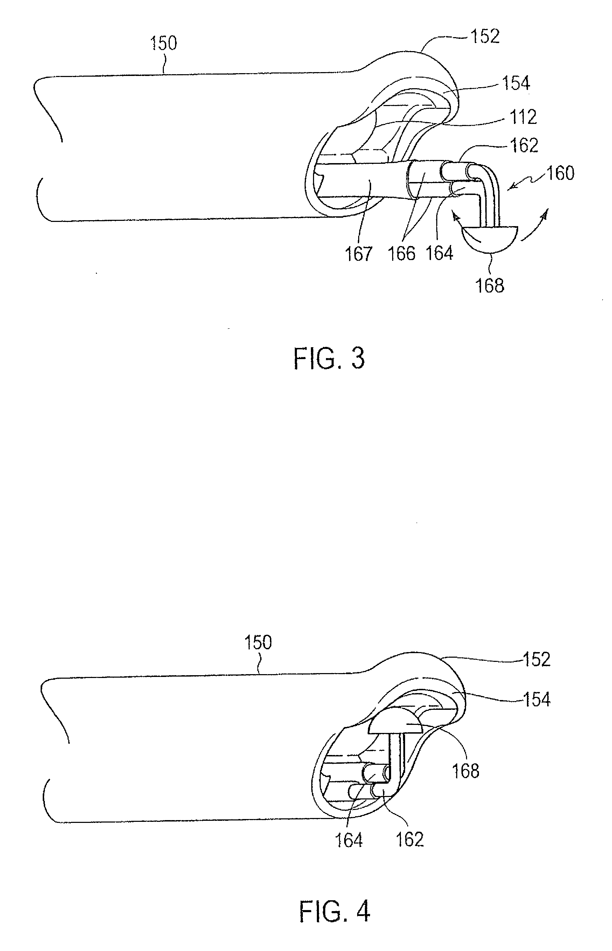

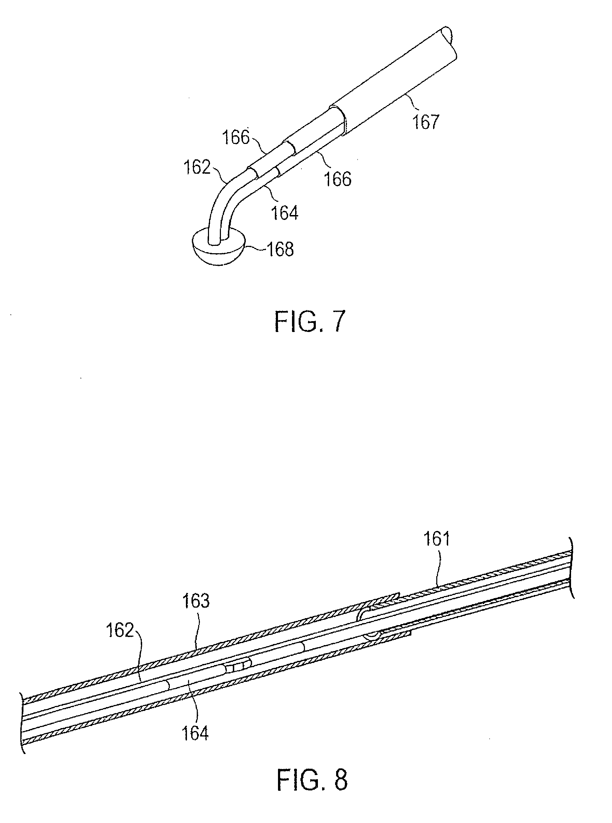

Sheath 150 can be smaller in cross-section than a typical resectoscope sheath, which is typically round in shape, and may have an oval shape. A suitable sheath is a laser sheath used with a continuous flow laser cystoscope, such as the Gyrus ACMI CLS-23SB, a 23 French Outer Sheath for a Continuous Flow Laser Resectoscope system available from Gyrus ACMI, Inc., of Southborough, Massachusetts. As better shown in Figs. 3-8, sheath 150 is connected to the connection part 120 at a proximal end thereof. A protruding distal tip portion 152 is provided at the distal end of the sheath, with an insulation layer 154 provided on at least the protruding distal tip portion 152. A through bore 156 extends longitudinally throughout the sheath for receiving the optics 116 and electrode assembly 160 therethrough. The phrases "proximal end" and "distal end" are not limited to the terminus of the sheath, but instead encompass the distal and proximal areas of the sheath.

The bipolar electrode assembly 160 includes active and return electrode wires 162, 164, respectively, each insulated by an insulation layer 166. The electrode wires 162, 164 are angled at their distal ends at a non-zero angle relative to the longitudinal axis of the sheath and connected to an electrode 168, such as the hemispherical button electrode shown. In this embodiment, the angle is a near perpendicular angle shown but may be an acute angle as shown in other embodiments. A protective sheath layer 167 surrounds the electrodes 162, 164.

The electrodes 162, 164 are provided within an external shaft, which includes a flexible shaft portion 161 and a rigid shaft portion 163, such as a metal shaft. In the illustrated embodiment, the rigid shaft portion 163 is provided near the distal end of the electrode assembly 160 within sheath 150 while the flexible shaft portion 161 is provided near the proximal end of the electrode assembly 160, including a portion extending through the working tool guide tube 126 and extending to fingertip control mechanism 130. The flexible shaft portion 161 allows for sufficient flexibility in the electrode assembly 160 for longitudinal and rotational motion within the curved working tool guide tube 126.

Power generator 140 can be a conventional RF generator and can be suitably controlled between on and off states by a foot control pedal 142. The RF generator is connected to electrode assembly 160 as known in the art.

To assemble the resection device, a surgeon inserts the electrode assembly 160 into the through bore 156 at the distal end of the sheath 150 until a proximal end of the electrode wires and flexible shaft 161 exit the working tool guide tube 126. The flexible shaft is then connected to the finger grip control mechanism 130 and the electrode wires 162, 164 are appropriately connected to RF generator 140. The finger grip control mechanism 130 is then suitably rotated and extended to position the distal end of the electrode assembly, including the electrode at an insertion/removal position discussed in more detail below.

As better shown in Fig. 4, the electrode assembly 160 is initially provided at an insertion/removal position where the electrode 168 and remainder of electrode wires 162, 164 are provided within the cross-section of the through bore 156 of the sheath. At this position, the electrode 168. is located directly opposed to the protruding tip 152 adjacent insulation layer 154. This allows for insertion or removal of the sheath from a patient, while protecting the electrode assembly 160 from short circuiting to the sheath due to the insulation layer 154. It is important to note that this insulation layer 154 is not provided in a conventional, laser sheath because the laser assembly is not subject to electrical shorting.

As better shown in Figs. 3-6, the electrode wires 162, 164 are oriented, at least near the distal end of sheath 150, to be closely adjacent one another and extend parallel with the longitudinal axis of sheath 150 and to be located one directly above the other in a stacked configuration. As better shown in Fig. 6, in various embodiments, the electrode wires 162, 164 are also in line with and immediately below optics 116. As compared with the split electrode wire configuration of the prior art (Fig. 17) where the electrode wires are provided on opposite sides of the optics, this can provide a reduced cross-sectional size of components within the sheath. This can allow for a reduction in the size of the sheath, minimizing the incision size necessary for the patient. Also, this orientation of the electrode assembly can reduce obstructions to visualization of the resection site.

As better shown in Fig. 3, the electrode assembly 160 upon insertion of the sheath 150 into the patient, may be repositioned to a resection position rotated away from the insertion position as shown. This movement is achieved by rotation of finger grip control mechanism 130. For example, a first resection position may be the position shown in Fig. 3, which is 180 degrees rotated from the insertion position. From this position, resection can occur through one or more of rotational (sweep) motion or longitudinal (push/pull) motion. Sweep motion is achieved by rotation of the finger grip control mechanism 130, which causes a rotational sweep motion about the longitudinal axis of the sheath 150 by the electrode 168 as shown by the directional arrows. This resection motion differs from conventional resection devices that rely on a longitudinal push/pull motion in line with the longitudinal axis of the sheath. However, the electrode assembly 160 and electrode 168 can also move in this direction as well under the control of the finger grip control mechanism 130.

In particular, once in the resection position, an operator can activate the RF generator 140, such as by depressing of the foot control pedal 142, to power the electrode 168 to cause resection of tissue. Because the inventive resection device 100 does not operate in "strokes" but instead may achieve free rotational or translational movement by manipulation of the finger grip control mechanism 130, resection can occur in a more continuous fashion, with a more continuous application of RF power to the electrode 168. This can achieve a more efficient resection through one or more of sweep and/or push/pull motion. Then, when resection is complete, the electrode assembly may be returned to the insertion/removal position shown in Fig. 4 by pulling of the finger grip control mechanism 130 rearward followed by rotation until the electrode is positioned opposed to the protruding insulated distal tip 152.

As can be seen from Figs. 1-2, finger grip control mechanism 130 is isolated and independent from other elements, including telescopic unit 110, connection part 120 and sheath 150. Therefore, compared to prior resection devices in which at least portions of the telescopic unit and sheath moved with movement of the control member, at least in rotary directions, movement by finger grip control mechanism 130 is isolated from and independent of movement of the telescopic unit 110.

As best shown in Figs. 1-2, finger grip control mechanism 130 is suitably sized and shaped with a cylindrical profile that allows it to be comfortably grabbed by a surgeon's fingertips within the palm of the surgeon's hand. The finger grip control mechanism may include a ribbed or otherwise discontinuous surface that achieves improved grip retention for enhancing control of the mechanism. By the finger grip control mechanism 130 being directly coupled to the electrode assembly 160, movement of the finger grip control mechanism 130 results in corresponding insertion/retraction or rotational movement of the electrode assembly in a continuous fashion. For instance, the surgeon may deploy the bipolar electrode by simply pushing the finger grip control mechanism 130 forward and twisting it 180 degrees so that the electrode 168 is extended from the sheath 150 and rotated to the resection position of Fig. 3. From here, the surgeon activates the electrode by control of the foot control pedal 142 of the RF generator 140 and resects tissue at a resection site by appropriate push/pull and twisting motion. Thus, compared to prior bipolar electrode resection devices, movement is not limited to a defined stroke in a single longitudinal direction. A preferable motion includes rotation of the electrode 180 about the longitudinal axis to achieve a rotary "sweep" resection. This can provide the surgeon with more flexibility and more intuitive control of the resection procedure by the bipolar electrode. Moreover, because the procedure can take place with a compound movement that is not limited to strokes, power from the RF generator 140 can be applied in a more continuous fashion, improving tissue resection efficiency. When resection is complete, the electrode assembly is again returned to the insertion/removal position shown in Fig. 4.

During resection, it is important to visualize the resection tissue. This is achieved by viewing the resection site with the optics 116 through the telescopic eyepiece 114. In conventional resection devices, such as those shown in Figs. 15-18, the electrode wires on the sides of the optics block only a lateral periphery of the resection site. However, because of the laterally spaced electrode wire positioning best shown in Fig. 17, the distal ends of the electrode wires 18A, 18B and electrode 18C provide a wide cross-sectional obstruction of view to the resection site. This can impede a surgeon's ability to properly visualize the resection operation.

The inventive electrode assembly 160 improves visualization of the resection site during a surgery procedure as best illustrated by a comparison of Figs. 6 and 17. As seen in Fig. 6, because of the over/under superimposed relationship of the electrode wires 162, 164, obstruction of the resection site in the field of view due to the electrode wires is greatly reduced compared to that of Fig. 17. When at the 6:00 o'clock position shown in Fig. 3 relative to optics 116, the electrode 168 can effectively hide its own shadow. However, the horizontal configuration of the prior art of Fig. 17 often results in a shadowing at the 3:00 o'clock and 9:00 o'clock positions that can cause difficulty in discerning and identifying important pathology as the surgeon inspects the resection site for suitable tissue areas for resection.

Additionally, by orienting the electrode wires vertically below the optics 116 rather than horizontally on both sides, peripheral viewing of the resection site is completely unobstructed. Thus, even using a hemispherical button electrode 168 as shown, the field of view perpendicular to the longitudinal axis of the sheath is less restricted than with the conventional electrode configuration.

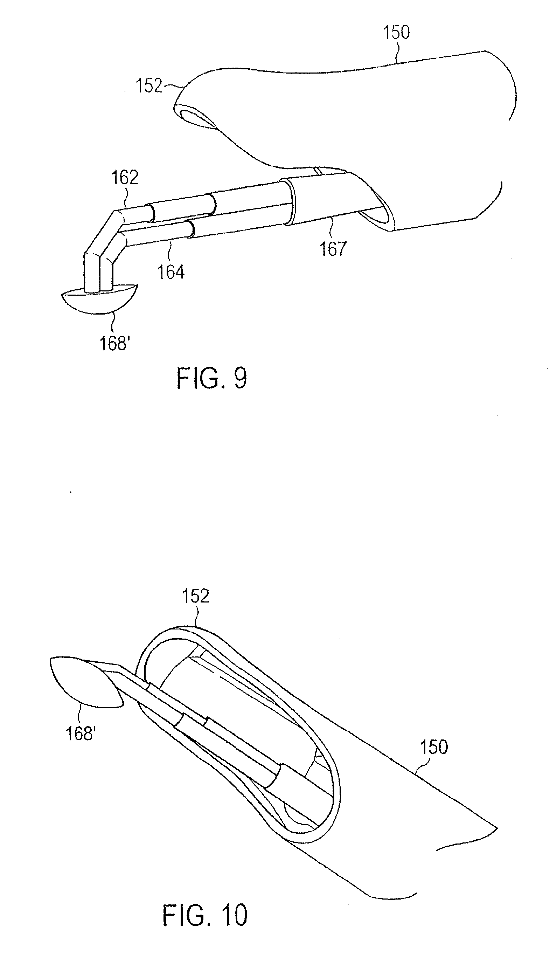

Further improvements in resection site visualization can be achieved through use of alternative electrode designs that provide a narrower obstruction to visualization while preferably retaining the capability of achieving sufficient resection speed. A first exemplary embodiment shown in Figs. 9-10 uses an ovoid shaped electrode 168' where the lateral dimension of the button electrode 168 is reduced to provide a slimmer profile for insertion and visualization. As in the previous embodiment, the ovoid button electrode 168' is oriented substantially perpendicular to the longitudinal axis. However, by providing approximately the same axial length of the button as the previous example (i.e., to achieve a relatively long longitudinal length relative to its width), the tissue cutting ability of this ovoid button electrode 168' can remain comparable to that of the hemispherical button electrode 168 when resection is achieved through a rotational "sweeping" motion. That is, when resection is achieved by sweeping the electrode rotationally about the longitudinal axis of the sheath, the ovoid button electrode provides 168' about the same overall contact size and thus can achieve a comparable resection of tissue. Additionally, when achieving resection through longitudinal movement of the electrode in the plane of the sheath, a narrower resection width of tissue will be vaporized. Thus, if desired, more precise resection can be achieved of smaller size. Thus, the ovoid configuration can allow for an endoscopic resection system with a smaller cross-sectional area to visualization while achieving similar or even better performance than a larger counterpart using a hemispherical button electrode.

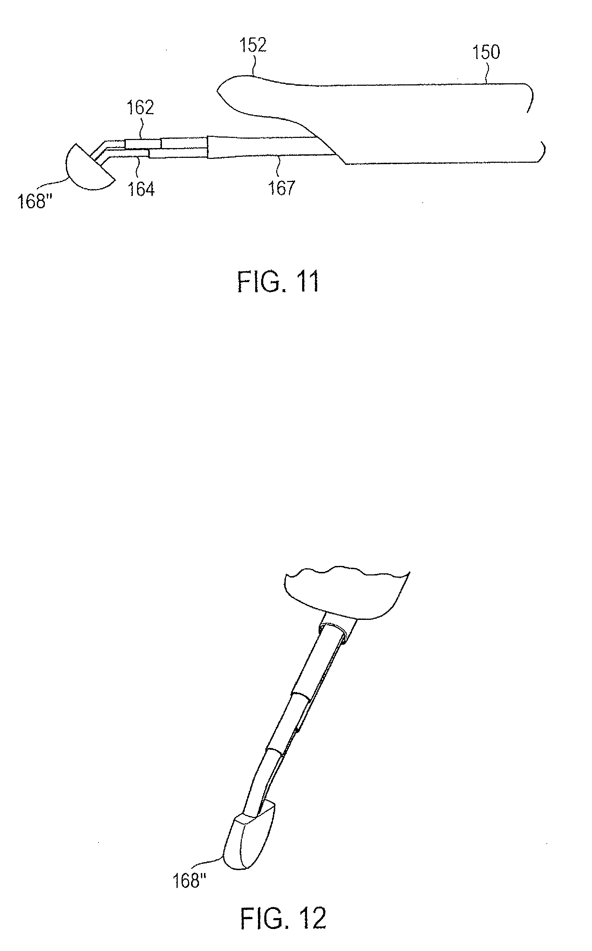

Another embodiment is shown in Figs. 11-12 and includes a narrow wedge electrode 168". This electrode may have the same center cross-section of the hemispherical button electrode 168, but has side portions removed to form the narrow wedge shape that improves visualization by providing a narrow cross-section to the field of view. However, because the cross-section in the rotary sweeping direction is comparable to that of the hemispherical button electrode 168, comparable resection can be achieved. Additionally, as in the previous example, because of the narrow width, resection using a longitudinal movement of the electrode may result in a narrowed resection width. The button electrode is not limited to a perpendicular angle as shown in previous examples. Instead, the button electrode can be provided at a non-zero acute angle, such as the angle of about 45 degrees shown which can allow resection directly in front of the electrode tip by sweeping the resectoscope tip, such as for use at the back wall of the bladder, while providing the same resection capability perpendicular to the axis of the electrode by rotating the electrode shaft as would be had with the button electrode or narrow ovoid electrode.

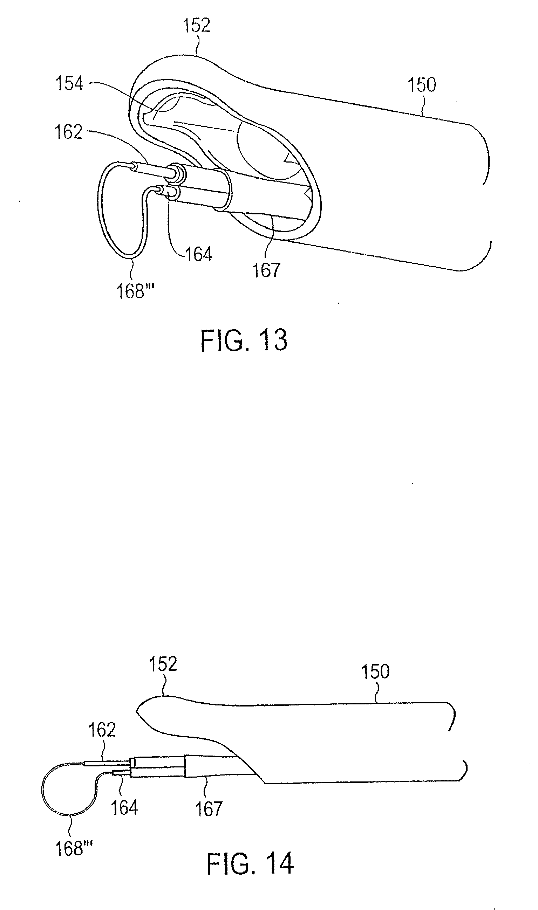

Another alternate configuration for the electrode is a loop electrode 168"' as shown in Figs. 13-14. However, whereas a typical loop electrode such as that shown in Fig. 17 extends lateral to the longitudinal axis of the sheath to achieve cutting action through a longitudinal pushing or pulling of the electrode assembly, this embodiment provides the loop electrode 168'" in line with the electrode wires 162, 162 and in line with the sheath. This embodiment further improves visualization by minimizing the obstructions to visualization of the resection site. Additionally, when used with the rotary sweep of the electrode assembly about the longitudinal axis of the sheath, as in previous examples, a lateral cutting action can occur with suitable tissue removal. Furthermore, since the loop continues up through the tip of the electrode, cutting tissue directly ahead of the electrode is facilitated, such as at the back wall of the bladder, by sweeping the scope tip either vertically or horizontally or at another angle, depending on the rotation orientation of the electrode loop, to achieve the best results.

## Claims

1. A resectoscope (100) that resects a resection tissue site, comprising:
a sheath (150) having a proximal end and a distal end defining a longitudinal through bore therebetween, the distal end having a protruding insulated distal tip;
a telescopic unit (110) comprising a telescope extending from the proximal end of the sheath and optics (116) extending from the telescope through the through bore and to the distal end of the sheath (150) where the optics provide visualization of the resection tissue site, the optics (116) extending longitudinally along the through bore;
a bipolar electrode assembly (160) extending from the proximal end of the sheath (150) through the through bore substantially parallel to the optics (116), and to the distal end of the sheath (150), the bipolar electrode assembly (160) including two electrode wires (162, 164) extending substantially parallel with the optics (116), the two electrode wires (162, 164) at least at the distal end of the sheath (150) being oriented one above the other and defining a longitudinal axis parallel to the longitudinal through bore, distal tips of the two bipolar electrode wires extending away from the optics at a non-zero angle relative to the longitudinal axis and being connected by an electrode (168) oriented in the plane of the longitudinal axis, the bipolar electrode assembly (160) further including a rigid shaft (163) surrounding at least an intermediate portion of the bipolar electrode wires within the sheath along the longitudinal axis and a flexible shaft (161) surrounding at least a proximal end of the bipolar electrode wires; and
a finger grip control mechanism (130) provided external to the sheath (150) and connected to the proximal end of the bipolar electrode wires (162, 164) with the flexible shaft (161) to manipulate movement of the bipolar electrode assembly (160) within a curved guide tube during resection by a sweeping rotary movement about the longitudinal axis, the rotary movement also including movement of the distal tips of the bipolar electrode wires (162, 164) between an insertion position where the distal tips are positioned within the sheath (150) opposing the protruding insulating distal tip and a resection position rotated away from the insertion position where the distal tips are oriented outside of the sheath (150), resection being achieved in the resection position by the sweeping rotary movement about the longitudinal axis,
wherein the finger grip control mechanism (130) is isolated from the telescopic unit such that rotation of the finger grip control mechanism is independent of rotation of the telescopic unit.

2. The resectoscope according to claim 1, wherein the electrode is a button electrode.

3. The resectoscope according to claim 2, wherein the button electrode is hemispherical in shape and extends substantially perpendicular to the longitudinal axis.

4. The resectoscope according to claim 1, wherein the electrode at the distal tips has a narrower cross-sectional profile perpendicular to the longitudinal axis than in the longitudinal axis to increase visualization while providing sufficient resection of tissue during resection by the sweeping rotary movement.

5. The resectoscope according to claim 3, wherein the button electrode has a narrow wedge shape oriented in the plane of the longitudinal axis.

6. The resectoscope according to claim 3, wherein the button electrode has a narrow ovoid shape oriented in the plane of the longitudinal axis.

7. The resectoscope according to claim 3, wherein the electrode is in the shape of a loop extending between the two electrode wires and oriented along the longitudinal axis for sweeping movement about the longitudinal axis as well as lateral movement across for a similar sweeping cut at an extreme tip of the electrode.

8. The resectoscope according to claim 1, wherein only the protruding distal tip of the sheath is insulated.

9. The resectoscope according to claim 1, wherein:
the sheath has an oval shape, and
the finger grip control mechanism has a rounded shape sized to be received within a hand of an operator for finger grip control, the finger grip control mechanism providing rotation of the bipolar electrode assembly by rotation of the finger grip control mechanism and translation of the bipolar electrode assembly by translation of the finger grip control mechanism.

10. The resectoscope according to claim 9, wherein the electrode has a narrow wedge shape oriented in the plane of the longitudinal axis.

11. The resectoscope according to claim 9, wherein the electrode has a narrow ovoid shape oriented in the plane of the longitudinal axis.

12. The resectoscope according to claim 9, wherein the electrode is in the shape of a loop extending between the two electrode wires and oriented along the longitudinal axis for sweeping movement about the longitudinal axis as well as lateral movement across for a similar sweeping cut at an extreme tip of the electrode.

13. The resectoscope according to claim 1, wherein:
the optics and bipolar electrode wires are oriented one above the other in the sheath along a common longitudinal plane,
the electrode has a narrower cross-sectional profile perpendicular to the longitudinal axis than in the longitudinal axis, and
the finger grip control mechanism has a rounded shape sized to be received within a hand of an operator for finger grip control, the finger grip control mechanism providing rotation of the bipolar electrode assembly by rotation of the finger grip control mechanism and translation of the bipolar electrode assembly by translation of the finger grip control mechanism.

## Patentansprüche

1. Resektoskop (100), das einen Geweberesektionsort reseziert, umfassend:
eine Umhüllung (150) mit einem proximalen Ende und einem distalen Ende, die zwischen sich eine Längsdurchgangsbohrung definieren, wobei das distale Ende eine vorspringende isolierte distale Spitze hat;
eine teleskopische Einheit (110) mit einem sich von dem proximalen Ende der Hülle erstreckenden Teleskop und mit einer sich von dem Teleskop durch die Durchgangsbohrung und zu dem distalen Ende der Hülle (150) erstreckenden Optik (116), wobei sich die Optik (116) in Längsrichtung entlang der Durchgangsbohrung erstreckt;
eine bipolare Elektrodenanordnung (160), die sich im Wesentlichen parallel zur Optik (116) von dem proximalen Ende der Umhüllung (150) durch die Durchgangsbohrung und zu dem distalen Ende der Umhüllung (150) erstreckt, wobei die bipolare Elektrodenanordnung (160) zwei Elektrodendrähte (162,164) hat, die sich im Wesentlichen parallel zur Optik (116) erstrecken, wobei die beiden Elektrodendrähte (162, 164) zumindest an dem distalen Ende der Umhüllung (150) übereinander orientiert sind und eine zu der Längsdurchgangsbohrung parallele Längsachse definieren, wobei sich die distalen vorderen Enden der beiden bipolaren Elektrodendrähte unter einem Nichtnullwinkel relativ zur Längsachse von der Optik weg erstrecken und durch eine in der Ebene der Längsachse orientierte Elektrode (168) verbunden sind, wobei die bipolare Elektrodenanordnung (160) ferner einen starren Schaft (163) umfasst, der zumindest einen Zwischenabschnitt der bipolaren Elektrodendrähte in der Umhüllung entlang der Längsachse umschließt, und einen flexiblen Schaft (161), der zumindest ein proximales Ende der bipolaren Elektrodendrähte umschließt; und
einen Fingergriff-Bedienmechanismus (130), der außen zur Umhüllung (150) vorgesehen und mit dem proximalen Ende der bipolaren Elektrodendrähte (162 164) mit dem flexiblen Schaft (161) verbunden ist, zum Manipulieren der Bewegung der bipolaren Elektrodenanordnung (160) während der Resektion in einem gekrümmten Führungsrohr durch eine auslandende Drehbewegung um die Längsachse, wobei die Drehbewegung auch eine Bewegung der distalen vorderen Enden der bipolaren Elektrodendrähte (162, 164) zwischen einer Einführposition, in der die distalen vorderen Enden in der Umhüllung (160) gegenüber der vorspringenden isolierenden distalen Spitze positioniert sind, und einer von der Einführposition weggedrehten Resektionsposition, in der die distalen vorderen Enden außerhalb der Umhüllung (150) orientiert sind, enthält, wobei die Resektion in der Resektionsposition erzielt wird durch die ausladende Drehbewegung um die Längsachse, wobei der Fingergriff-Bedienmechanismus (130) von der teleskopischen Einheit derart isoliert ist, dass eine Drehung des Fingergriff-Bedienmechanismus von der Drehung der teleskopischen Einheit unabhängig ist.

2. Resektoskop nach Anspruch 1, wobei die Elektrode eine Knopfelektrode ist.

3. Resektoskop nach Anspruch 2, wobei die Knopfelektrode halbkugelförmig ist und sich im Wesentlichen senkrecht zur Längsachse erstreckt.

4. Resektoskop nach Anspruch 1, wobei die Elektrode an den distalen vorderen Enden senkrecht zur Längsachse ein schmaleres Querschnittprofil als in der Längsachse hat, um die Visualisierung zu verbessern und dabei gleichzeitig für eine ausreichende Geweberesektion durch die ausladende Drehbewegung während der Resektion zu sorgen.

5. Resektoskop nach Anspruch 3, wobei die Knopfelektrode eine schmale Keilform hat, die in der Ebene der Längsachse orientiert ist.

6. Resektoskop nach Anspruch 3, wobei die Knopfelektrode eine schmale Eiform hat, die in der Ebene der Längsachse orientiert ist.

7. Resektoskop nach Anspruch 3, wobei die Elektrode eine Schlingenform hat, die sich zwischen den Elektrodendrähten erstreckt und entlang der Längsachse orientiert ist, für eine ausladende Bewegung um die Längsachse sowie eine seitliche Bewegung kreuzweise, für einen ähnlich schweifenden Schnitt an einer äußersten Spitze der Elektrode.

8. Resektoskop nach Anspruch 1, wobei nur das distale vorspringende vordere Ende der Umhüllung isoliert ist.

9. Resektoskop nach Anspruch 1, wobei:
die Umhüllung eine ovale Form hat und
der Fingergriff-Bedienmechanismus eine gerundete Form hat, die in der Größe derart bemessen ist, dass sie zur Fingergriffbedienung in der Hand eines Operateurs aufgenommen werden kann, wobei der Fingergriff-Bedienmechanismus für eine Drehung der bipolaren Elektrodenanordnung sorgt, indem der Fingergriff-Bedienmechanismus gedreht wird, und für eine Translation der bipolaren Elektrodenanordnung, indem der Fingergriff-Bedienmechanismus translatorisch bewegt wird.

10. Resektoskop nach Anspruch 9, wobei die Elektrode eine schmale Keilform hat, die in der Ebene der Längsachse orientiert ist.

11. Resektoskop nach Anspruch 9, wobei die Elektrode eine schmale Eiform hat, die in der Ebene der Längsachse orientiert ist.

12. Resektoskop nach Anspruch 9, wobei die Elektrode eine Schlingenform hat, die sich zwischen den beiden Elektrodendrähten erstreckt und entlang der Längsachse orientiert ist, für eine ausladende Bewegung um die Längsachse sowie eine seitliche Bewegung kreuzweise, für einen ähnlich schweifenden Schnitt an einer äußersten Spitze der Elektrode.

13. Resektoskop nach Anspruch 1, wobei:
die Optik und die bipolaren Elektrodendrähte in der Umhüllung entlang einer gemeinsamen Längsebene übereinander orientiert sind, wobei die Elektrode senkrecht zur Längsachse ein schmaleres Querschnittprofil als in der Längsachse hat und
wobei der Fingergriff-Bedienmechanismus eine gerundete Form hat, die in der Größe derart bemessen ist, dass sie zur Fingergriffbedienung in der Hand eines Operateurs aufgenommen werden kann, wobei der Fingergriff-Bedienmechanismus für eine Drehung der bipolaren Elektrodenanordnung sorgt, indem der Fingergriff-Bedienmechanismus gedreht wird, und für eine Translation der bipolaren Elektrodenanordnung, indem der Fingergriff-Bedienmechanismus translatorisch bewegt wird.

## Revendications

1. Résectoscope (100) qui résèque un site tissulaire de résection, comprenant :
une gaine (150) ayant une extrémité proximale et une extrémité distale définissant un trou de passage longitudinal entre elles, l'extrémité distale ayant une pointe distale isolée en saillie ;
une unité télescopique (110) comprenant un télescope s'étendant à partir de l'extrémité proximale de la gaine et une optique (116) s'étendant à partir du télescope à travers le trou de passage et jusqu'à l'extrémité distale de la gaine (150) où l'optique fournit une visualisation du site tissulaire de résection, l'optique (116) s'étendant longitudinalement le long du trou de passage ;
un ensemble d'électrode bipolaire (160) s'étendant de l'extrémité proximale de la gaine (150) à travers le trou de passage sensiblement parallèlement à l'optique (116) et jusqu'à l'extrémité distale de la gaine (150), l'ensemble d'électrode bipolaire (160) comprenant deux fils d'électrode (162, 164) s'étendant sensiblement parallèlement à l'optique (116), les deux fils d'électrode (162, 164), au moins à l'extrémité distale de la gaine (150), étant orientés l'un au-dessus de l'autre et définissant un axe longitudinal parallèle au trou de passage longitudinal, des pointes distales des deux fils d'électrode bipolaire s'étendant en s'éloignant de l'optique avec un angle non nul part rapport à l'axe longitudinal et étant connectés par une électrode (168) orientée dans le plan de l'axe longitudinal, l'ensemble d'électrode bipolaire (160) comprenant en outre une tige rigide (163) entourant au moins une partie intermédiaire des fils d'électrode bipolaire à l'intérieur de la gaine le long de l'axe longitudinal et une tige flexible (161) entourant au moins une extrémité proximale des fils d'électrode bipolaire ; et
un mécanisme de commande à prise pour le doigt (130) disposé à l'extérieur de la gaine (150) et connecté à l'extrémité proximale des fils d'électrode bipolaire (162, 164) avec la tige flexible (161) pour manipuler le mouvement de l'ensemble d'électrode bipolaire (160) à l'intérieur d'un tube de guidage courbé durant la résection par un mouvement de rotation à balayage autour de l'axe longitudinal, le mouvement de rotation comprenant également le mouvement des pointes distales des fils d'électrode bipolaire (162, 164) entre une position d'insertion dans laquelle les pointes distales sont positionnées à l'intérieur de la gaine (150) opposées à la pointe distale isolante en saillie et une position de résection pivotée éloignée de la position d'insertion dans laquelle les pointes distales sont orientées à l'extérieur de la gaine (150), la résection étant obtenue dans la position de résection par le mouvement de rotation à balayage autour de l'axe longitudinal,
dans lequel le mécanisme de commande à prise pour le doigt (130) est isolé de l'unité télescopique de manière que la rotation du mécanisme de commande à prise pour le doigt soit indépendante de la rotation de l'unité télescopique.

2. Résectoscope selon la revendication 1, dans lequel l'électrode est une électrode à bouton.

3. Résectoscope selon la revendication 2, dans lequel l'électrode à bouton est de forme hémisphérique et s'étend sensiblement perpendiculairement à l'axe longitudinal.

4. Résectoscope selon la revendication 1, dans lequel l'électrode au niveau des pointes distales a un profil en coupe transversale perpendiculairement à l'axe longitudinal plus étroit que dans l'axe longitudinal pour augmenter la visualisation tout en fournissant une résection suffisante de tissu durant la résection par le mouvement de rotation à balayage.

5. Résectoscope selon la revendication 3, dans lequel l'électrode à bouton a une forme de coin étroit orientée dans le plan de l'axe longitudinal.

6. Résectoscope selon la revendication 3, dans lequel l'électrode à bouton a une forme ovoïde étroite orientée dans le plan de l'axe longitudinal.

7. Résectoscope selon la revendication 3, dans lequel l'électrode est sous la forme d'une boucle s'étendant entre les deux fils d'électrode et orientée suivant l'axe longitudinal pour un mouvement de balayage autour de l'axe longitudinal ainsi qu'un mouvement latéral en travers pour une coupe de balayage similaire au niveau d'une pointe d'extrémité de l'électrode.

8. Résectoscope selon la revendication 1, dans lequel seule la pointe distale en saillie de la gaine est isolée.

9. Résectoscope selon la revendication 1, dans lequel :
la gaine a une forme ovale, et
le mécanisme de commande à prise pour le doigt a une forme arrondie dimensionné pour être reçu dans une main d'un opérateur pour la commande à prise pour le doigt, le mécanisme de commande à prise pour le doigt fournissant une rotation de l'ensemble d'électrode bipolaire par rotation du mécanisme de commande à prise pour le doigt et une translation de l'ensemble d'électrode bipolaire par translation du mécanisme de commande à prise pour le doigt.

10. Résectoscope selon la revendication 9, dans lequel l'électrode a une forme de coin étroit orientée dans le plan de l'axe longitudinal.

11. Résectoscope selon la revendication 9, dans lequel l'électrode a une forme ovoïde étroite orientée dans le plan de l'axe longitudinal.

12. Résectoscope selon la revendication 9, dans lequel l'électrode est sous la forme d'une boucle s'étendant entre les deux fils d'électrode et orientée suivant l'axe longitudinal pour un mouvement de balayage autour de l'axe longitudinal ainsi qu'un mouvement latéral en travers pour une coupe de balayage similaire au niveau d'une pointe d'extrémité de l'électrode.

13. Résectoscope selon la revendication 1, dans lequel :
l'optique et les fils d'électrode bipolaire sont orientés l'un au-dessus de l'autre dans la gaine le long d'un plan longitudinal commun,
l'électrode a un profil en coupe transversale perpendiculairement à l'axe longitudinal plus étroit que dans l'axe longitudinal, et
le mécanisme de commande à prise pour le doigt a une forme arrondie dimensionné pour être reçu dans une main d'un opérateur pour la commande à prise pour le doigt, le mécanisme de commande à prise pour le doigt fournissant une rotation de l'ensemble d'électrode bipolaire par rotation du mécanisme de commande à prise pour le doigt et une translation de l'ensemble d'électrode bipolaire par translation du mécanisme de commande à prise pour le doigt.
